# EUROPEAN PATENT SPECIFICATION

(11) **EP 1 655 697 B1**
(45) Date of publication and mention of the grant of the patent: **13.05.2009**
(21) Application number: 03818102.0
(22) Date of filing: 13.08.2003
(51) Int. Cl.: G06K 9/00

(54) **PERSONAL IDENTIFICATION DEVICE**
PERSÖNLICHE IDENTIFIKATIONSEINRICHTUNG
DISPOSITIF D'IDENTIFICATION PERSONNELLE

(43) Date of publication of application: 10.05.2006
(73) Proprietor: Hitachi, Ltd., Tokyo 100-8280 (JP)
(72) Inventor: MATSUMURA, Takafumi, Tokyo 100-8220 (JP); OSAKA, Ichiro, Tokyo 100-8220 (JP); KASHIMURA, Yuichi, Tokyo 100-8220 (JP); MIYATAKE, Takafumi, Tokyo 100-8220 (JP); NAGASAKA, Akio, Tokyo 100-8220 (JP); MIURA, Naoto, Tokyo 100-8220 (JP)
(74) Representative: Beetz & Partner
(86) International application number: PCT/JP2003/010292
(87) International publication number: WO 2005/017828

(56) References cited:
- EP-A2- 1 187 055
- WO-A-01/57784
- WO-A1-99/43258
- WO-A1-02/069244
- JP-A- 4 088 586
- JP-A- 10 003 532
- JP-A- 2002 157 586
- JP-A- 2003 030 632
- US-A- 5 177 802
- US-A- 5 177 802
- US-A1- 2002 067 845
- US-A1- 2003 016 345

## Description

### Technical Field

The present invention relates to a personal identification device for identifying a person based on information of a living body, and more particularly to a personal identification device for identifying a person based on a blood vessel pattern of a finger.

### Background Art

An expectation for personal identification techniques is increased for the purpose of safe management of property and information. Particularly, a living-body identification technique utilizing a part of the living body as a key receives attention because of having a less risk of fraudulent practice with loss, theft, etc. than the known method using a password or a cryptographic key. Regarding the living-body identification technique, studies have been conducted on various methods utilizing fingerprints, faces, irises, blood vessel patterns of hands and fingers, etc. Among them, the identification method utilizing the blood vessel patterns of fingers is advantageous in neither reminding a person of crime unlike the case of utilizing fingerprints, nor requiring light to be directly illuminated to an eyeball unlike the case of utilizing irises, thus causing the person to feel a less psychological repulsion.

Another advantage is that, because the identification method utilizing the blood vessel patterns of fingers handles the feature of the interior of a living body rather than the surface of the living body, forgery is difficult.

JP-A-2003-30632 discloses one example of the identification method utilizing the blood vessel patterns of fingers. In the disclosed example, a light source for emitting a near-infrared ray is prepared and a camera is installed in relation to face the light source. An optical filter allowing passage of only the wavelength of a near-infrared range is mounted to the camera. In personal identification, a finger is placed between the camera and the light source to pick up a finger image. Because components in blood absorb the near-infrared ray well, the near-infrared ray does not pass through blood vessels and the blood vessels appear as shades in the image. After processing the feature of a picked-up blood vessel pattern on the basis of a parameter, the processed parameter is checked for a match with a previously registered parameter for the personal identification.

US 2003/0016345 A1 describes a finger identification apparatus having a light source opposite to a camera. A finger is placed inbetween the light source and the camera and a picture is taken and analyzed to identify a person. This apparatus is quite bulky due to the use of a camera and due to the fact that the whole finger has to be illuminated at the time of picture taking.

US 5177802 shows a fingerprint input apparatus using light reflected from a finger to analyze the pattern of a fingerprint. To identify a person the fingerprint is fairly unsave, as the pattern may be damaged or hurt by accident.

### Disclosure of the Invention

### Problems to be Solved by the Invention

In the device disclosed in JP-A-2003-30632, however, a lens is mounted on the camera for picking up an image, and therefore downsizing of the device is difficult to realize. One solution for the downsizing is, for example, to employ a wide-angle lens, but it has a limitation in widening the field. Also, the use of a wide-angle lens tends to cause image distortion. The position of a finger of which image has been picked up for registration often differs from the position of the finger of which image is picked up for identification. For example, when the finger is positioned at the end of an image area at the time of picking up the finger image for identification while the finger is positioned at the center of the image area at the time of picking up the finger image for registration, the finger image picked up for identification is distorted. In that case, therefore, the blood vessel pattern obtained for identification is determined as being not the same as the blood vessel pattern obtained for registration.

Meanwhile, there is a demand for mounting a personal identification device in a place having a limited mounting space, such as in cars and cell phones.

Accordingly, an object of the present invention is to provide, in a personal identification device utilizing information of a living body, such as a blood vessel pattern means capable of downsizing the device. Another object is to provide means capable of reducing consumption of power. Still another object is to provide means for reducing the influence of rotation of a finger about an axis of the finger. Still another object is to provide means for reducing the influence of dirt, dust, etc. which are attached to a light receiving element row for capturing an image and to provide pictures with an increased contrast.

These objects are accomplished with a personal identification device as claimed in claim 1.

Dependent claims are directed on features of preferred embodiments of the invention.

### Brief Description of the Drawings

Fig. 1 shows an external appearance of a personal identification device according to the present invention.
Fig. 2 shows one example of a blood vessel pattern of finger.
Fig. 3 shows a first example of the configuration of the personal identification device according to the present invention.
Fig. 4 shows one example of an infrared source drive circuit according to the present invention.
Fig. 5 is an explanatory view for explaining an infrared ray reflected by the finger.
Fig. 6 shows a first example of a light receiving element row according to the present invention.
Fig. 7 is a flowchart for explaining a personal identification process in the personal identification device according to the present invention.
Fig. 8 shows a second example of the configuration of the personal identification device according to the present invention.
Fig. 9 shows a third example of the configuration of the personal identification device according to the present invention.
Fig. 10 is an explanatory view for explaining the position of an infrared source in the personal identification device according to the present invention.
Fig: 11 shows a fourth example of the personal identification device according to the present invention.
Fig. 12 shows a fifth example of the personal identification device according to the present invention.
Fig. 13 is an explanatory view for explaining the position of the light receiving element row in the personal identification device according to the present invention.
Fig. 14 shows a sixth example of the personal identification device according to the present invention.
Fig. 15 shows an external appearance of a seventh example of the personal identification device according to the present invention.
Fig. 16 shows an eighth example of the personal identification device according to the present invention.

### Reference Numerals

1...identification device, 3...external input/output line, 10...light receiving element row, 12...infrared source, 13...signal processing unit, 14...input/output terminal, 15...microcomputer, 16...interface circuit, 17...power supply circuit, 20...casing, 21...cavity, 22...ceiling portion, 23...bottom portion, 31...bottom portion, 32...frame member, 40...support member, 41...roof portion, 42...bottom portion, 43...support portion, 200...finger, and 201...blood vessel pattern.

### Best Mode for Carrying Out the Invention

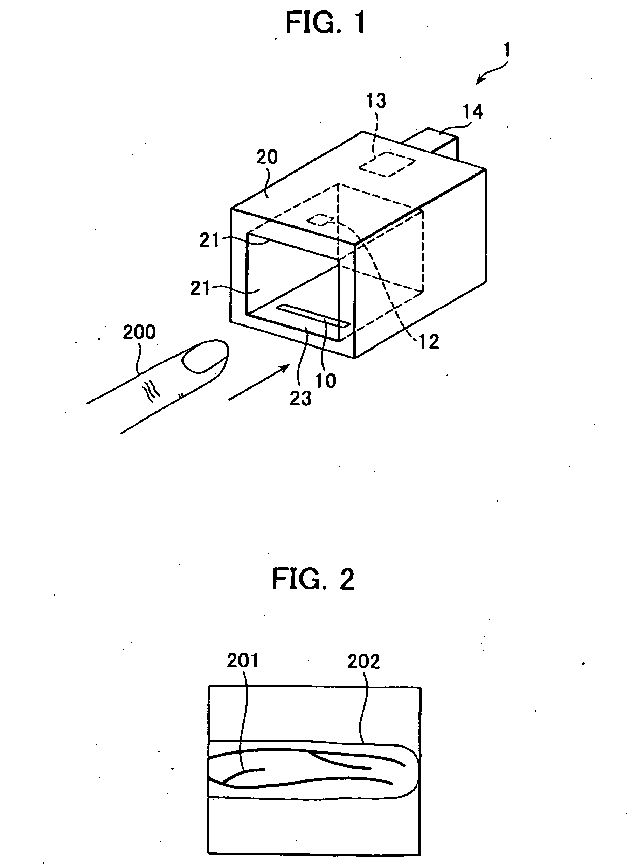

The general structure of an identification device 1 according to the present invention, which utilizes blood vessel patterns of fingers, will be described below with reference to Fig. 1. The identification device 1 has a casing 20 substantially in a parallelepiped shape, and a cavity 21 allowing insertion of a finger 200 is formed in the front side of the casing. An infrared source 12 for emitting an infrared ray is mounted to a ceiling portion 22 on the inner side of the casing 20, and a light receiving element row 10 containing a plurality of light receiving elements arranged in line is mounted to a bottom portion 23 on the inner side of the casing. The identification device 1 further includes a signal processing unit 13 for processing signals from the light receiving element row 10 and recognizing a blood vessel pattern, and an input/output terminal 14 for communicating information with respect to external equipment via an input/output line 3.

The infrared source 12 and the light receiving element row 10 are disposed near an entrance of the cavity 21 in the casing 20 so as to face each other. The light receiving element row 10 is arranged to lie perpendicularly to the direction in which the finger 200 is inserted. When a fore end of the finger 200 is inserted in the cavity 21 in the casing 20, an infrared ray is illuminated from the infrared source 12 to the surface of back of the finger 200. A part of the infrared ray passes through the finger 200, comes out to the exterior from the belly of the finger, and is received by the light receiving element row 10. When the finger 200 is moved toward the innermost end of the cavity 21 in the casing 20 while passing over the light receiving element row 10, an infrared image of the finger is obtained by the light receiving element row 10. Because the infrared ray is absorbed by blood flowing through blood vessels, the infrared image obtained by the light receiving element row 10 contains shades attributable to the blood vessels and exhibits a brightness distribution.

Fig. 2 shows an image of the blood vessel pattern obtained by the light receiving element row 10. The horizontal axis indicates relative displacements of the finger with respect to the light receiving element row, and the vertical axis indicates outputs of the light receiving element row. As shown, a blood vessel pattern 201 is represented by dark lines along with an image 202 of the finger.

The signal processing unit 13 checks the blood vessel pattern obtained by the light receiving element row for a match with a previously registered pattern, and the check result is outputted via the input/output terminal 14.

The identification device 1 of the illustrated example basically needs one infrared source 12 as a light source and is not required to use a plurality of infrared sources unlike the related art. Hence, lower consumption of power can be realized.

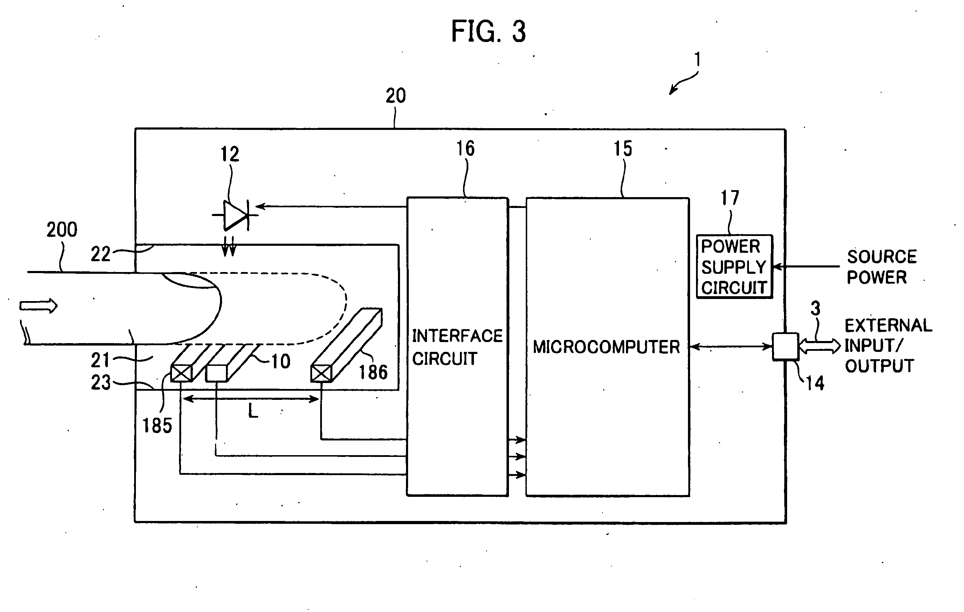

A first example of the identification device 1 according to the present invention will be described with reference to Fig. 3. The identification device 1 comprises an infrared source 12, a light receiving element row 10, finger detecting devices 185, 186, an interface circuit 16, a microcomputer 15, a power supply circuit 17, and an input/output terminal 14. These components are mounted in the casing 20.

The infrared source 12 is mounted to the ceiling portion 22 on the inner side of the casing 20, and the light receiving element row 10 and the finger detecting devices 185, 186 are mounted to the bottom portion 23 on the inner side of the casing 20. The infrared source 12 and the light receiving element row 10 are arranged near the entrance of the cavity 21 in the casing 20. One 185 of the finger detecting devices 185, 186 is arranged near the entrance of the cavity 21, while the other 186 is arranged at an inner position within the cavity. The light receiving element row 10 and the finger detecting devices 185, 186 are each arranged, as shown, to lie perpendicularly to the direction in which the finger 200 is inserted.

In the illustrated example, the finger detecting device 185 on the entrance side and the light receiving element row 10 are disposed separately from each other, but those components may be constituted as one common device.

The infrared source 12 can be constituted by, e.g., a molded LED (Light Emitting Diode) utilizing a GaAs-based device. The light receiving element row 10 contains a plurality of light receiving elements arranged in line, and each of the light receiving element converts the received light to an electrical signal.

The finger detecting devices 185, 186 are used to determine the presence of the finger 200. An optical device such as a photodiode, an electrostatic capacitance device, an electrode, or the like can be used as each of the finger detecting devices 185, 186.

When the finger 200 is inserted in the cavity 21, the finger detecting device 185 on the entrance side first detects the finger. The microcomputer 15 then starts to take in outputs from the light receiving element row 10. When the finger 200 is inserted into the more inner side of the cavity 21, the finger detecting device 186 on the inner side detects the finger. The microcomputer 15 then stops the taking-in of the outputs from the light receiving element row 10. By measuring a distance L between the two finger detecting devices 185 and 186 and a time t from the start to end of the taking-in of the outputs, an average moving speed v of the finger can be determined. From the finger moving speed, it is possible to determine a pitch at which the outputs from the light receiving element row 10 are taken in.

The microcomputer 15 executes control of the amount of light (infrared ray) emitted from the infrared source 12, processing of respective finger detection signals from the finger detecting devices 185, 186 and light intensity signals from the light receiving element row 10, and a personal identification process based on the blood vessel pattern. The interface circuit 16 connects the infrared source 12, the light receiving element row 10, and the finger detecting devices 185, 186 to the microcomputer 15. The power supply circuit 17 supplies power necessary for each component of the identification device 1.

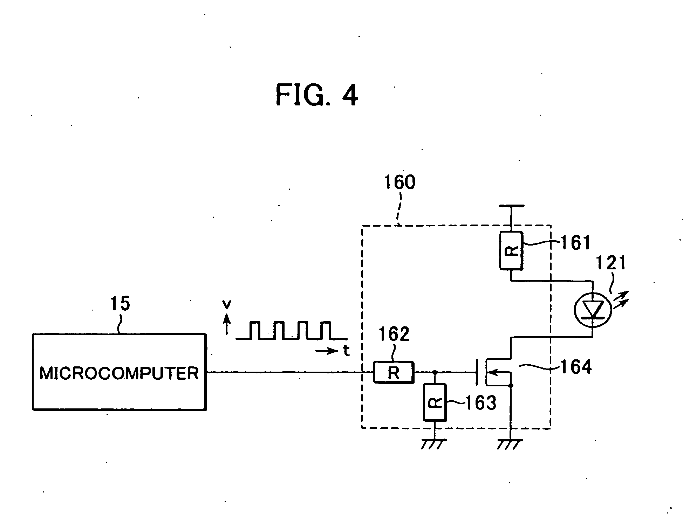

An example of a drive circuit for driving an infrared source 12 will be described with reference to Fig. 4. A drive circuit 160 of this embodiment comprises resistances 161, 162 and 163, and an Nch-MOS transistor 164. The microcomputer 15 outputs a PWM (Pulse Width Modulation) signal capable of executing duty ratio control and drives the Nch-MOS transistor 164. The driving frequency is set to the range of several tens to several hundreds kHz. When the time during which each light receiving element of the light receiving element row 10 shown in Fig. 3 integrates the received light is sufficiently longer than the driving cycle of the Nch-MOS transistor 164, each light receiving element of the light receiving element row 10 operates as the low-band passage type. In other words, only DC-component light is received and an image containing no appreciable unevenness is obtained. In addition to the PWM signal, a DC voltage signal, a PAM (Pulse Amplitude Modulation) signal, or the like can also be used as the driving signal.

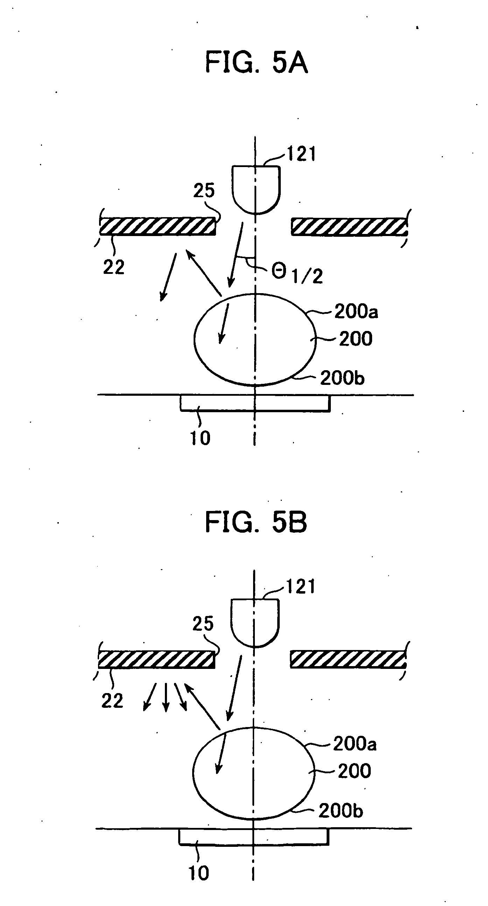

The positional relationships among the infrared source 121, the finger 200, and the light receiving element row 10 will be described with reference to Fig. 5. In this example, the infrared ray from the infrared source 121 passes through a window 25 formed in the ceiling portion 22 of the casing and is illuminated to the back 200a of the finger 200. The finger 200 is positioned such that the illuminated infrared ray advances toward the surface of the finger back 200a with a directivity characteristic with a half-value angle θ_{1/2}· A part of the infrared ray illuminated to the surface of the finger back 200a passes through the finger 200, comes out to the exterior from the surface of the finger belly 200b, and is received by the light receiving element row 10. The remaining part of the infrared ray illuminated to the surface of the finger back 200a is reflected by the surface of the finger back 200a. A part of the reflected infrared ray is further reflected by the ceiling portion 22 of the casing.

In this example, the surface of the ceiling portion 22 is preferably smooth as shown in Fig. 5(a). When the surface of the ceiling portion 22 is smooth, the infrared ray reflected by the ceiling portion 22 advances in a direction away from the finger and does not enter the light receiving element row 10. Accordingly, the reflected infrared ray causes no noise.

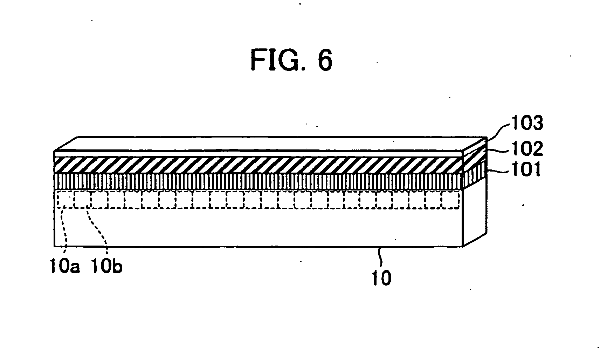

If the surface of the ceiling portion 22 is not smooth as shown in Fig. 5(b), the infrared ray reflected by the ceiling portion 22 advances in various directions, and a part of the reflected infrared ray reaches the light receiving element row 10. Such a part of the infrared ray causes noise in the captured image of the blood vessel pattern of the finger. An example of the structure of the light receiving element row 10 will be described with reference to Fig. 6. The light receiving element row 10 comprises a protective film 103 disposed on the row surface, a first filter 102 disposed under the protective film 103, a second filter 101 disposed under the first filter 102, and a plurality of light receiving elements 10a, 10b, ... which are arranged in line under the second filter 101 and convert the infrared ray to electrical signals.

The first filter 102 cuts visible light, but allows transmission of only an infrared component through it. On the other hand, each of the light receiving elements 10a, 10b has a characteristic exhibiting no sensitivity for wavelengths longer than a certain one. Thus, based on the transmission characteristic of the filter 102 and the wavelength sensitivity characteristic of the light receiving elements 10a, 10b, ..., each of the light receiving elements 10a, 10b has sensitivity for a certain width of wavelength. As a result, the blood vessel pattern can be imaged with good contrast.

The second filter 101 allows transmission of only a component of the incident light, which perpendicularly enters the second filter. The reason why the second filter 101 is disposed is as follows. The infrared ray is absorbed by the blood vessels of the finger 200, while it is scattered inside the finger and has components advancing in all directions. Accordingly, the light receiving element 10 receives the infrared components having various incident angles. If the second filter 101 is not disposed, the light receiving elements 10a, 10b, ... detect the sum of the infrared components outgoing from a wide area over the surface of the finger belly, thus resulting lower contrast of the blood vessel pattern. In this example, the provision of the second filter 101 enables the light receiving element to receive only the component of the incident light, which perpendicularly enters it.

The light receiving elements 10a, 10b can be formed of, e.g., photodiodes integrated on a silicon substrate. The center-to-center interval between adjacent two of the light receiving elements 10a, 10b, ... is decided depending on the thickness of the blood vessels of the finger. By setting the interval to the range of about 0.02 mm to 0.5 mm, the image of the blood vessel pattern sufficient for the personal identification can be obtained.

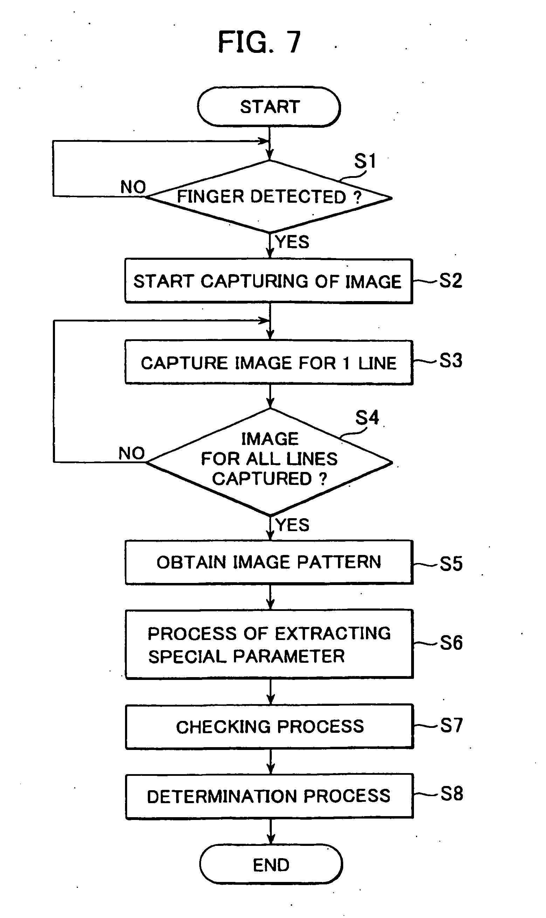

Fig. 7 shows another example of the light receiving element row 10. This example employs a lens array 104 instead of the second filter 101 in Fig. 6. The lens array 104 collects the infrared rays scattered by the finger surface and contributes to obtaining an image with higher contrast.

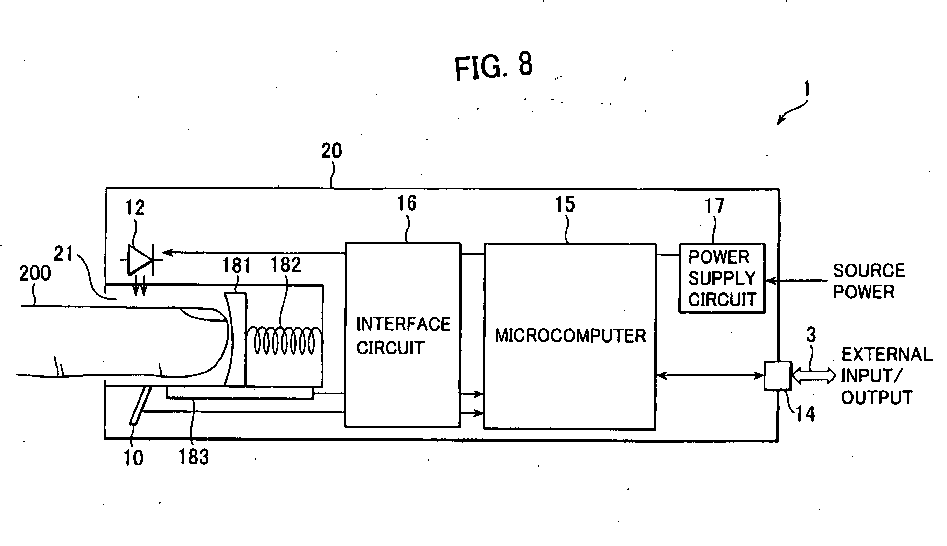

The operation of the identification process will be described with reference to Fig. 8. The identification process is executed in accordance with a program stored in a memory of the microcomputer 15. First, in step S1, it is determined whether the finger detecting device 185 on the entrance side has detected the finger 200. If the finger is not detected, the process returns to step S1, and if detected, the process goes to step S2.

In step S2, the microcomputer 15 starts to capture the image of the blood vessel pattern. In step S3, the microcomputer 15 takes in signals for one line of the image from the light receiving element row 10. Based on the light intensity signals from the light receiving element row 10, the microcomputer 15 controls the amount of light emitted from the infrared source 12 so that the contrast between an area corresponding to the blood vessel pattern and the remaining area is optimized. In step S4, the microcomputer 15 determines whether signals for all lines of the image have been taken in from the light receiving element row 10.

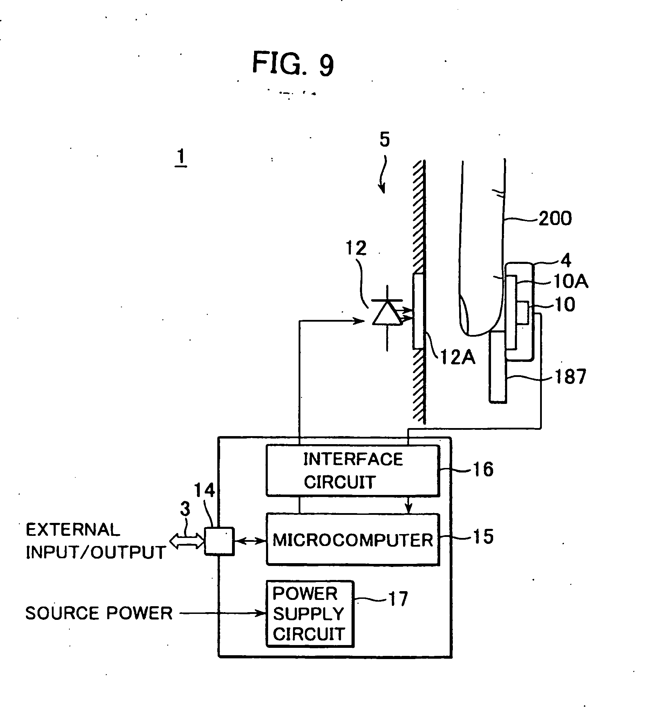

In the example of Fig. 3, whether the signals for all the lines have been taken in can be determined with the detection of the finger by the finger detecting device 186 on the inner side. Alternatively, as shown in another example of Fig. 9, the amount of movement of a button may be detected to determine whether the signals for all the lines have been taken in.

If the image of all the lines has not yet been captured, the process returns to step S3, and if the image of all the lines has been captured, the process goes to step S5.

In step S5, the microcomputer 15 produces the blood vessel pattern. To that end, the moving speed of the finger is first computed based on the time from the start to end of the image capturing and the distance over which the finger has moved. Then, the pitch of capturing the image is computed from the moving speed of the finger. Based on the capturing time and the capturing pitch, the absolute position of the captured data along the axial direction of the finger is determined and a two-dimensional distribution of brightness of the image is obtained.

In step S6, the microcomputer executes a process for extracting a feature parameter of the blood vessel pattern of the finger. In step S7, the extracted feature parameter is compared with one or more previously feature parameters to check for a match between them. When a particular person is to be identified as in the case of authenticating the user of a car, a single feature parameter is prepared. When a plurality of qualified persons are to be identified as in the case of authenticating persons qualified to enter a particular building, a plurality of feature parameters are prepared.

In step S8, the check result is determined. If the check result shows a proper match, a predetermined output is issued to indicate that the relevant person is registered. If no match is found between the feature parameters, an output is issued to indicate that the relevant person is not registered or that the check has failed.

A second example of the identification device according to the present invention will be described with reference to Fig. 9. The identification device 1 of this example comprises an infrared source 12, a light receiving element row 10, a button 181, a spring 182, a linear encoder 183, an interface circuit 16, a microcomputer 15, a power supply circuit 17, and an input/output terminal 14. These components are mounted in a casing 20. The identification device of this example differs from the identification device shown in Fig. 3 in that the button 181, the spring 182, and the linear encoder 183 are provided instead of the finger detecting devices 185, 186. The operations of the button 181, the spring 182, and the linear encoder 183 will be described below.

When the finger 200 is inserted in the cavity 21, the finger strikes against the button 181. When the finger 200 is inserted into the more inner side of the cavity 21, the button 181 is moved correspondingly. The position of the button 181 is detected by the linear encoder 183. When the linear encoder 183 detects the start of movement of the button 181, the microcomputer 15 starts reading of the light intensity signals from the light receiving element row 10. The linear encoder 183 successively detects the position of the button 181 and sends the detected position to the microcomputer 15. Based on the position of the button 181 from the linear encoder 183 and the light intensity signals from the light receiving element row 10, the microcomputer 15 obtains a two-dimensional distribution of brightness of the image shown in Fig. 2. When the finger 200 is withdrawn out of the cavity, the button 181 is returned to the original position by the spring 182.

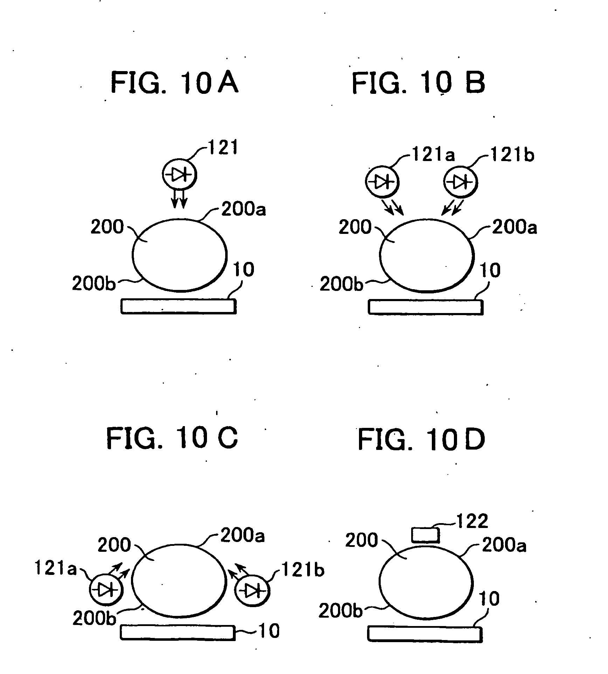

A third example of the identification device according to the present invention will be described with reference to Fig. 10. Fig. 10 shows the state where the identification device of this example is disposed in a car door. The identification device of this example comprises an infrared source 12, a light receiving element row 10, a shutter 187, an interface circuit 16, a microcomputer 15, a power supply circuit 17, and an input/output terminal 14.

The infrared source 12 is disposed in a car door 5 and is covered with a transparent protective film 12A. The light receiving element row 10 is disposed on the inner side of a door handle 4 to face the infrared source 12. A recess is formed in the door handle 4, and the light receiving element row 10 is arranged in the recess. The light receiving element row 10 is also covered with a transparent protective film 10A. Further, a movable shutter 187 is disposed on the inner side of the door handle 4. The movement of the shutter 187 is detected by a linear encoder (not shown). When the shutter 187 is moved by the finger 200, the linear encoder generates a trigger. In response to the trigger from the linear encoder, the microcomputer 15 starts to capture the image.

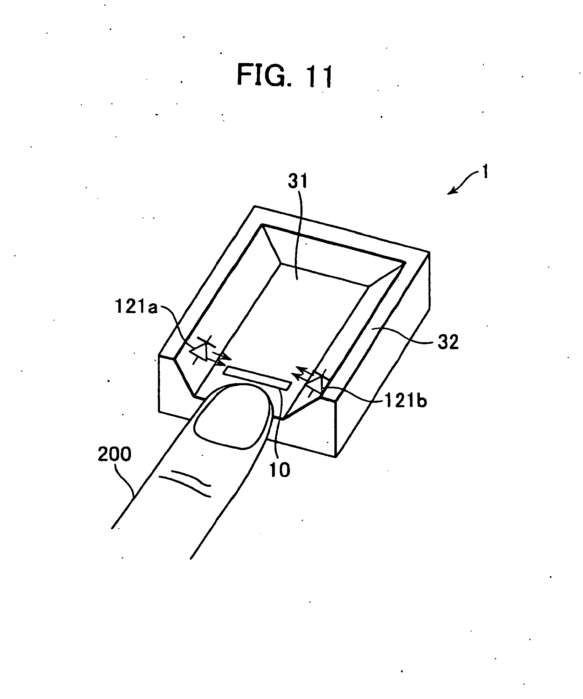

The positional relationship between the infrared source and the light receiving element row 10 will be described with reference to Figs. 11, 12 and 13. In an example shown in Fig. 11(a), an infrared source 121 is disposed on the side to face the finger back 200a, and the light receiving element row 10 is disposed on the side to face the finger belly 200b. This arrangement is the same as that shown in Figs. 1 and 3. As another example, however, two infrared sources 121a, 121b may be disposed in spaced positions, as shown in Fig. 11(b), such that respective infrared rays are illuminated downward obliquely from above at different angels from each other. This arrangement is advantageous in increasing the maximum amount of light.

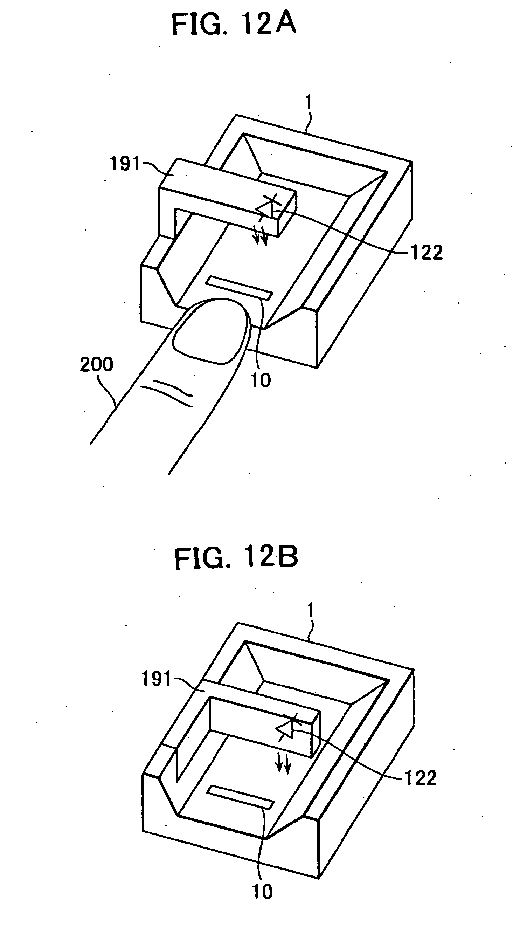

As still another example shown in Fig. 11(c), two infrared sources 121a, 121b may be disposed on both sides of the finger such that respective infrared rays are illuminated upward obliquely from relatively low positions near the finger belly 200b at different angels from each other. In this case, the light receiving element row 10 receives reflected or scattered components of the illuminated infrared rays. Fig. 12 shows an example of the identification device in which two infrared sources 121a, 121b are disposed so as to illuminate respective infrared rays upward obliquely from relatively low positions near the finger belly 200b. The identification device of this example comprises a bottom portion 31 and a frame member 32 disposed at three sides of the former in surrounding relation. The infrared sources 121a, 121b are mounted on the inner side of the frame member 32. The light receiving element row 10 is disposed on the bottom portion 31. With this example, since the casing 20 shown in Fig. 1 is not used, the finger back 200a is not hidden by the infrared sources, thus enabling the relevant person to feel openness when he or she places the finger 200 on the bottom portion for personal identification. In addition, downsizing of the identification device 1 can be realized.

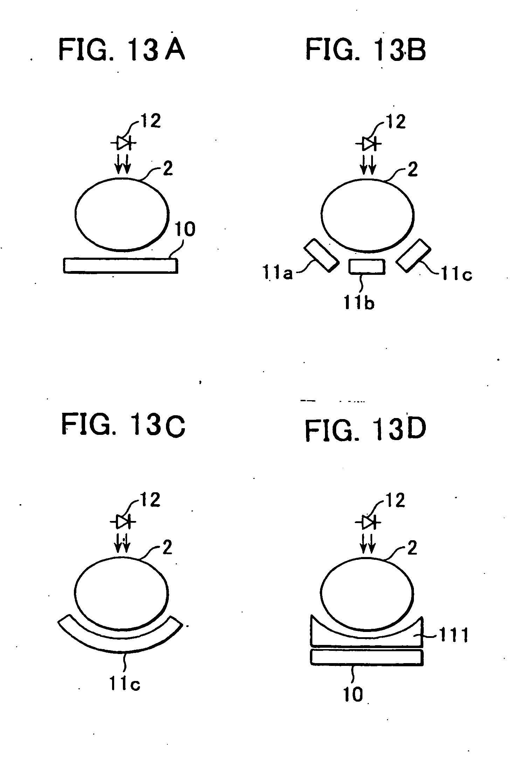

In still another example shown in Fig. 11(d), a chip type infrared source 122 is employed. With the use of the chip type infrared source, the thickness of a portion including the infrared source can be reduced. In an example shown in Fig. 13, the chip type infrared source 122 is mounted to a foldable cantilevered beam 191. When the identification device is used, the cantilevered beam 191 is turned to stand as shown in Fig. 13(a), and when it is not used, the cantilevered beam 191 is folded as shown in Fig. 13(b). The identification device of this example has a smaller size and is suitable for carrying with.

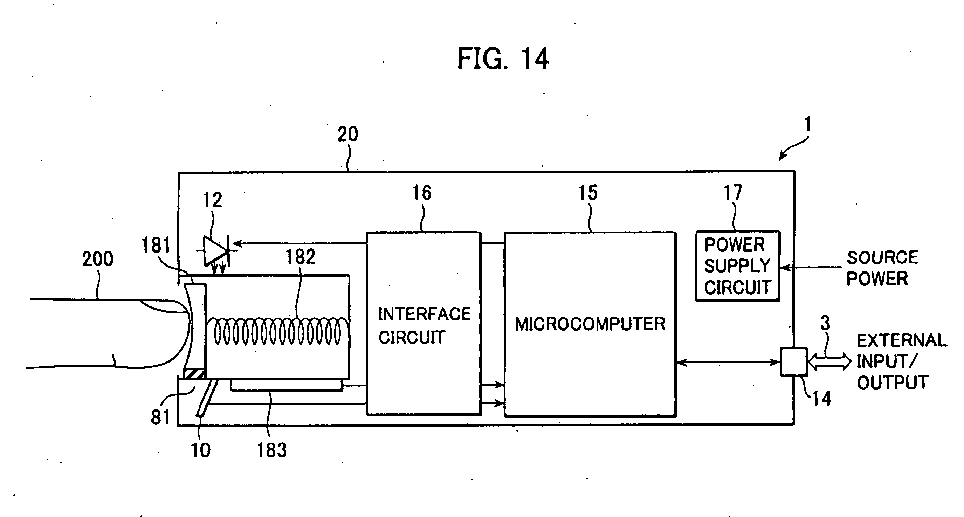

The form of the light receiving element row 10 will be described with reference to Fig. 14. In an example shown in Fig. 14(a), the light receiving element row 10 has a linear shape. This form is the same as that in the example shown in Figs. 1 and 3. Alternatively, as shown in Fig. 14(b), a plurality of light receiving element rows 11a, 11b, 11c, ... may be arranged in surrounding relation to the finger. As another example, a light receiving element row 11d having a curved shape may be used as shown in Fig. 14(c). As still another example, a concave lens 111 may be arranged on the light receiving element row 10 as shown in Fig. 14(d).

In the examples shown in Figs. 14(b), 14(c) and 14(d), the light receiving element row 10 is arranged to extend in the circumferential direction. Accordingly, even when the finger 200 is rotated about its axis while the finger is inserted, an image distortion caused due to the finger rotation about the axis is suppressed small although position correction is required. As a result, an error of the blood vessel pattern can be reduced. Stated another way, even if the finger is rotated about the axis, the obtained data can be checked for a match with the registered data.

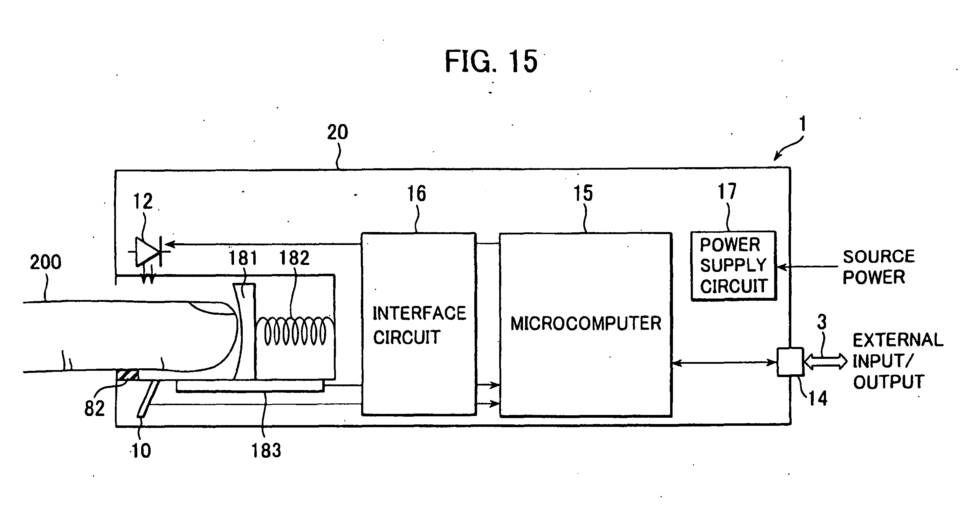

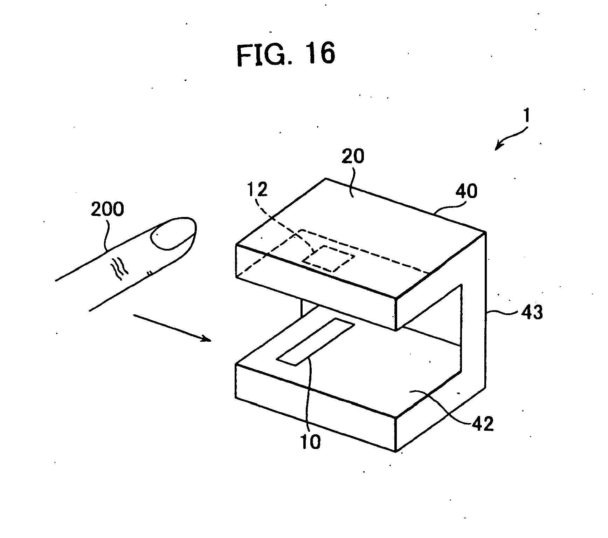

Figs. 15 and 16 show modifications of the second example of the identification device, shown in Fig. 9, according to the present invention. In the example of Fig. 15, a brush 81 is disposed at an end surface of the button 181. When the button 181 is moved, the brush 81 is also moved to clean the surface of the light receiving element row 10. Dust, dirt and other contaminations attached on the surface of the light receiving element row 10 are thus removed. It is therefore possible to reduce image variations caused by the contaminations on the surface of the light receiving element row 10. Also, in the example of Fig. 16, a brush 82 is disposed at the entrance of the cavity. When the finger 200 is inserted in the cavity, the finger surface is cleaned by the brush 82, and dust, dirt and other contaminations attached on the surface of the finger are removed. It is therefore possible to reduce image variations caused by the contaminations on the surface of the finger 200. Further, the provision of the brush 81 is effective in preventing dust and dirt from entering the interior of the cavity, thereby keeping the surface of the light receiving element row 10 from being contaminated.

In the above-described personal identification devices according to the present invention, the blood vessel pattern of the hand finger is used as data to be checked. However, the blood vessel pattern of the back or palm of a hand may be used as the data to be checked instead of the blood vessel pattern of the finger. When the blood vessel pattern of a hand is used for check, the device size is increased, but the device structure is basically the same as that of the above-described identification device.

While the present invention has been described in connection with the examples, it is easily understood by those skilled in the art that the present invention is not limited to the above-described examples, but it can be modified in various ways without departing from the scope of the invention defined in the claims.

## Claims

1. A personal identification device (1) comprising an infrared source (12) for illuminating an infrared ray to a target to be identified, and a light receiving element row disposed to face each other containing a plurality of light receiving elements (10) arranged in line which receive the infrared ray illuminated from said infrared source (12), said target (200) to be identified is insertable between said infrared light source (12) and said light receiving element row (10) in a direction perpendicular to a long side of said light receiving element row, wherein said light receiving element row is provided with a filter member (101) allowing transmission of only a component of incident light, which substantially perpendicularly enters said light receiving element row, and wherein when said target is relatively scanned with respect to said light receiving element row, a two-dimensional image representing a blood vessel pattern (201) of said target (200) to be identified is produced from outputs of said light receiving element row and relative displacement information of said target to be identified, thereby performing personal identification based on the produced image.

2. The personal identification device (1) according to Claim 1, wherein said target (200) to be identified is a human hand or finger.

3. The personal identification device (1) according to any one of Claims 1 and 2, wherein a position detecting device (185, 186) for detecting a position of said target (200) to be identified is disposed, and said two-dimensional image of said target (200) to be identified is produced from the outputs of said light receiving element row and position information from said position detecting device (185, 186).

4. The personal identification device (1) according to any one of Claims 1 to 3, wherein an identified-target detecting device (185, 186) for detecting the presence or absence of said target (200) to be identified is disposed in a position away from said light receiving element row.

5. The personal identification device (1) according to Claim 4, wherein said identified-target detecting device (185, 186) is disposed in plural, a speed of said target (200) to be identified is computed from a difference between passage times of one end of said target (200) to be identified, which are detected by said plurality of identified-target detecting devices (185, 186), and distance correction of said image in a scan direction is performed based on the speed of said target (200) to be identified.

6. The personal identification device (1) according to Claim 4, wherein the speed of said target (200) to be identified is computed from a difference between passage times of one end of said target (200) to be identified, which are detected by said light receiving element row and said identified-target detecting device (185, 186) disposed one or in plural, and a distance correction of said image in a scan direction is performed based on the speed of said target (200) to be identified.

7. The personal identification device (1) according to any one of Claims 1 to 6, wherein said light receiving element row contains a plurality of light receiving elements (11) arrayed along a curved line.

8. The personal identification device (1) according to any one of Claims 2 to 6, wherein said light receiving element row comprises a plurality of light receiving element rows (11a, 11b, 11c), and said plurality of light receiving element rows (11a, 11b, 11c) are arranged along a curved line.

9. The personal identification device (1) according to any one of Claims 6 or 7, wherein an interval between two adjacent light receiving elements in said light receiving element row is from 0.02 mm to 0.5 mm.

10. A personal identification device (1) according to one of claims 1 to 9, comprising a casing (20), and a light source (12) and a light receiving element row (10) both disposed in said casing (20), said device (1) operating such that when a finger (200) is inserted in said casing (20), the light from said light source (12) is illuminated to the finger (200), the light having passed through the finger (200) is detected by said light receiving element row (10), and a blood vessel pattern (201) of the finger (200) is produced from outputs of said light receiving element row (10), thereby performing personal identification based on the produced blood vessel pattern (201), wherein said casing (20) has a cavity (21) in which the finger (200) is inserted, and said light receiving element row (10) is arranged perpendicularly to a direction of depth of said cavity (21).

11. A personal identification device (1) according to any of claims 1 to 10, comprising a C-shaped support member (40) including a first member (41), a second member (42) and a third member (43) for connecting said first (41) and second members (42) to each other, an infrared source (12) mounted to said first member (41), and a light receiving element row (10) mounted to said second member (42), said device (1) operating such that when a finger (200) is scanned over said light receiving element row (10), an infrared ray from said infrared source (12) is illuminated to the finger (200), the infrared ray having passed through the finger (200) is detected by said light receiving element row (10), and a blood vessel pattern (201) of the finger (200) is produced from outputs of said light receiving element row (10), thereby performing personal identification based on the produced blood vessel pattern.

12. A personal identification device (1) according to claim 10 comprising a bottom member, a frame member disposed to surround said bottom member from three sides thereof, said infrared source being mounted to said frame member, and said light receiving element row (10) being mounted to said bottom member, said device (1) operating such that when a finger is scanned over said light receiving element row (10), an infrared ray from said infrared source (12) is illuminated to the finger, the infrared ray having passed through the finger (200) is detected by said light receiving element row, and a blood vessel pattern (201) of the finger (200) is produced from outputs of said light receiving element row (10), thereby performing personal identification based on the produced blood vessel pattern (201).

13. A personal identification device (1) according to claim 10, said device operating such that when a finger (200) is inserted in said casing, an infrared ray from said infrared source (12) is illuminated to the finger (200), the infrared ray having passed through the finger is detected by said light receiving element row (10), and a blood vessel pattern (201) of the finger (200) is produced from outputs of said light receiving element row (10), thereby performing personal identification based on the produced blood vessel pattern (201), wherein said casing has a smooth inner surface to prevent a part of the infrared ray from said infrared source, which has been reflected by the finger, from entering said light receiving element row (10).

14. The personal identification device (1) according to any one of Claims 1 to 13, wherein personal identification is performed by comparing a previously registered feature parameter and a feature parameter of an image obtained from the outputs of said light receiving element row (10).

15. The personal identification device (1) according to Claim 3, wherein said position detecting device is provided with a button (181) capable of being pushed by the finger (200), cleaning means (183) is mounted to said button (181), and a surface of said light receiving element row (10) is cleaned with scan of said button (181).

## Patentansprüche

1. Persönliche Identifikationseinrichtung (1) mit einer Infrarotquelle (12) zum Abstrahlen eines Infrarotstrahls auf ein zu identifizierendes Ziel und einer Lichtempfangselementreihe, die einander zugewandt angeordnet sind, enthaltend mehrere Lichtempfangselemente (10), die in einer Linie angeordnet sind, welche den von der Infrarotquelle (12) abgestrahlten Infrarotstrahl empfangen, das zu identifizierende Ziel (200) kann zwischen die Infrarotlichtquelle (12) und die Lichtempfangselementreihe (10) in einer Richtung eingeführt werden, die zu einer Längsseite der Lichtempfangselementreihe senkrecht ist, wobei die Lichtempfangselementreihe mit einem Filterelement (101) versehen ist, das die Übertragung von nur einem Bestandteil von einfallendem Licht gestattet, welches im Wesentlichen senkrecht in die Lichtempfangselementreihe eintritt, und wobei, wenn das Ziel bezüglich der Lichtempfangselementreihe relativ abgetastet wird, ein zweidimensionales Bild, das ein Blutgefäßmuster (201) des zu identifizierenden Ziels (200) darstellt, aus Ausgaben der Lichtempfangselementreihe und Relativvrschiebungsinformation des zu identifizierenden Ziels erzeugt wird, wodurch auf der Grundlage des erzeugten Bilds eine persönliche Identifizierung durchgeführt wird.

2. Persönliche Identifikationseinrichtung (1) nach Anspruch 1, wobei das zu identifizierende Ziel (200) eine menschliche Hand oder ein menschlicher Finger ist.

3. Persönliche Identifikationseinrichtung (1) nach irgendeinem der Ansprüche 1 und 2, wobei eine Positionserfassungsvorrichtung (185, 186) zum Erfassen einer Position des zu identifizierenden Ziels (200) angeordnet ist und das zweidimensionale Bild des zu identifizierenden Ziels (200) aus den Ausgaben der Lichtempfangselementreihe und Positionsinformation von der Positionserfassungsvorrichtung (185, 186) erzeugt wird.

4. Persönliche Identifikationseinrichtung (1) nach irgendeinem der Ansprüche 1 bis 3, wobei eine Identifiziertes-Ziel-Erfassungsvorrichtung (185, 186) zum Erfassen der Anwesenheit oder Abwesenheit des zu identifizierenden Ziels (200) in einer von der Lichtempfangselementreihe entfernten Position angeordnet ist.

5. Persönliche Identifikationseinrichtung (1) nach Anspruch 4, wobei die Identifiziertes-Ziel-Erfassungsvorrichtung (185, 186) zu mehreren angeordnet ist, die Geschwindigkeit des zu identifizierenden Ziels (200) aus der Differenz zwischen Durchgangszeiten von einem Ende des zu identifizierenden Ziels (200), die durch die mehreren Identifiziertes-Ziel-Erfassungsvorrichtungen (185, 186) erfasst werden, berechnet wird und eine Entfemungskorrektur des Bilds in Abtastrichtung auf der Grundlage der Geschwindigkeit des zu identifizierenden Ziels (200) durchgeführt wird.

6. Persönliche Identifikationseinrichtung (1) nach Anspruch 4, wobei die Geschwindigkeit des zu identifizierenden Ziels (200) aus der Differenz zwischen Durchgangszeiten von einem Ende des zu identifizierenden Ziels (200), die durch die Lichtempfangselementreihe und die einzeln oder zu mehreren angeordnete Identifiziertes-Ziel-Erfassungsvorrichtung (185, 186) erfasst werden, berechnet wird und eine Entfernungskorrektur des Bilds in Abtastrichtung auf der Grundlage der Geschwindigkeit des zu identifizierenden Ziels (200) durchgeführt wird.

7. Persönliche Identifikationseinrichtung (1) nach irgendeinem der Ansprüche 1 bis 6, wobei die Lichtempfangselementreihe mehrere Lichtempfangselemente (11) enthält, die entlang einer gekrümmten Linie angeordnet sind.

8. Persönliche Identifikationseinrichtung (1) nach irgendeinem der Ansprüche 2 bis 6, wobei die Lichtempfangselementreihe mehrere Lichtempfangselementreihen (11a, 11b, 11c) umfasst und die mehreren Lichtempfangselementreihen (11a, 11b, 11c) entlang einer gekrümmten Linie angeordnet sind.

9. Persönliche Identifikationseinrichtung (1) nach irgendeinem der Ansprüche 6 oder 7, wobei ein Abstand zwischen zwei benachbarten Lichtempfangselementen in der Lichtempfangselementreihe 0,02 mm bis 0,5 mm beträgt.

10. Persönliche Identifikationseinrichtung (1) nach einem der Ansprüche 1 bis 9, mit einem Gehäuse (20) und einer Lichtquelle (12) und einer Lichtempfangselementreihe (10), die beide in dem Gehäuse (20) angeordnet sind, wobei die Einrichtung (1) so arbeitet, dass, wenn ein Finger (200) in das Gehäuse (20) eingeführt wird, das Licht von der Lichtquelle (12) auf den Finger (200) abgestrahlt, das durch den Finger (200) hindurchgegangene Licht von der Lichtempfangselementreihe (10) erfasst und ein Blutgefäßmuster (201) des Fingers (200) aus Ausgaben der Lichtempfangselementreihe (10) erzeugt wird, wodurch eine persönliche Identifikation auf der Grundlage des erzeugten Blutgefäßmusters (201) durchgeführt wird, wobei das Gehäuse (20) einen Hohlraum (21) aufweist, in den der Finger (200) eingeführt wird, und die Lichtempfangselementreihe (10) senkrecht zur Tiefenrichtung des Hohlraums (21) angeordnet ist.

11. Persönliche Identifikationseinrichtung (1) nach irgendeinem der Ansprüche 1 bis 10, mit einem C-förmigen Halteelement (40), das ein erstes Element (41), ein zweites Element (42) und ein drittes Element (43) zum miteinander Verbinden des ersten (41) und zweiten (42) Elements beinhaltet, einer an dem ersten Element (41) angebrachten Infrarotquelle (12) und einer an dem zweiten Element (42) angebrachten Lichtempfangselementreihe (10), wobei die Einrichtung (1) so arbeitet, dass, wenn ein Finger (200) über der Lichtempfangselementreihe (10) abgetastet wird, ein Infrarotstrahl von der Infrarotquelle (12) auf den Finger (200) abgestrahlt, der durch den Finger (200) hindurchgegangene Infrarotstrahl von der Lichtempfangselementreihe (10) erfasst und ein Blutgefäßmuster (201) des Fingers (200) aus Ausgaben der Lichtempfangselementreihe (10) erzeugt wird, wodurch eine persönliche Identifikation auf der Grundlage des erzeugten Blutgefäßmusters durchgeführt wird.

12. Persönliche Identifikationseinrichtung (1) nach Anspruch 10, mit einem unteren Element, einem Rahmenelement, das so angeordnet ist, dass es das untere Element von drei Seiten umgibt, wobei die Infrarotquelle an dem Rahmenelement angebracht und die Lichtempfangselementreihe (10) an dem unteren Element angebracht ist, wobei die Einrichtung (1) so arbeitet, dass, wenn ein Finger über der Lichtempfangselementreihe (10) abgetastet wird, ein Infrarotstrahl von der Infrarotquelle (12) auf den Finger abgestrahlt, der durch den Finger (200) hindurchgegangene Infrarotstrahl von der Lichtempfangselementreihe erfasst und ein Blutgefäßmuster (201) des Fingers (200) aus Ausgaben der Lichtempfangselementreihe (10) erzeugt wird, wodurch eine persönliche Identifikation auf der Grundlage des erzeugten Blutgefäßmusters (201) durchgeführt wird.

13. Persönliche Identifikationseinrichtung (1) nach Anspruch 10, wobei die Einrichtung so arbeitet, dass, wenn ein Finger (200) in das Gehäuse eingeführt wird, ein Infrarotstrahl von der Infrarotquelle (12) auf den Finger (200) abgestrahlt, der durch den Finger hindurchgegangene Infrarotstrahl von der Lichtempfangselementreihe (10) erfasst und ein Blutgefäßmuster (201) des Fingers (200) aus Ausgaben der Lichtempfangselementreihe (10) erzeugt wird, wodurch eine persönliche Identifikation auf der Grundlage des erzeugten Blutgefäßmusters (201) durchgeführt wird, wobei das Gehäuse eine glatte Innenoberfläche aufweist, um einen Teil des Infrarotstrahls von der Infrarotquelle, der von dem Finger reflektiert worden ist, am Eintritt in die Lichtempfangselementreihe (10) zu hindern.

14. Persönliche Identifikationseinrichtung (1) nach irgendeinem der Ansprüche 1 bis 13, wobei eine persönliche Identifikation durch Vergleichen eines zuvor registrierten Merkmalsparameters und eines Merkmalsparameters eines Bilds, das von den Ausgaben der Lichtempfangselementreihe (10) erhalten wurde, durchgeführt wird.

15. Persönliche Identifikationseinrichtung (1) nach Anspruch 3, wobei die Positionserfassungsvorrichtung mit einem Knopf (181) versehen ist, welcher von dem Finger (200) gedrückt werden kann, eine Reinigungseinrichtung (183) an dem Knopf (181) angebracht ist und die Oberfläche der Lichtempfangselementreihe (10) mit dem Abtasten des Knopfs (181) gereinigt wird.

## Revendications

1. Dispositif d'identification personnelle (1) comprenant une source infrarouge (12) permettant d'émettre un rayon infrarouge en direction d'une cible à identifier, et une rangée d'éléments récepteurs de lumière disposés de manière à se faire face contenant une pluralité d'éléments récepteurs de lumière (10) disposés en ligne qui reçoivent le rayon infrarouge émis par ladite source infrarouge (12), ladite cible (200) à identifier peut être insérée entre ladite source lumineuse infrarouge (12) et ladite rangée d'éléments récepteurs de lumière (10) dans une direction perpendiculaire à une longueur de ladite rangée d'éléments récepteurs de lumière, dans lequel ladite rangée d'éléments récepteurs de lumière est munie d'un élément de filtre (101) permettant la transmission d'uniquement une composante de lumière incidente, qui pénètre de manière sensiblement perpendiculaire dans ladite rangée d'éléments récepteurs de lumière, et dans lequel quand ladite cible subit un balayage relatif par rapport à ladite rangée d'éléments récepteurs de lumière, une image bidimensionnelle représentant un motif de vaisseaux sanguins (201) de ladite cible (200) à identifier est produite à partir des signaux de sortie de ladite rangée d'éléments récepteurs de lumière et des informations de déplacement relatif de ladite cible à identifier, ce qui permet l'identification personnelle sur la base de l'image produite.

2. Le dispositif d'identification personnelle (1) selon la revendication 1, dans lequel ladite cible (200) à identifier est une main ou un doigt humain.

3. Le dispositif d'identification personnelle (1) selon l'une quelconque des revendications 1 et 2, dans lequel un dispositif de détection de position (185, 186) permettant de détecter une position de ladite cible (200) à identifier est disposé, et ladite image bidimensionnelle de ladite cible (200) à identifier est produite à partir des signaux de sortie de ladite rangée d'éléments récepteurs de lumière et des informations de position provenant dudit dispositif de détection de position (185, 186).

4. Le dispositif d'identification personnelle (1) selon l'une quelconque des revendications 1 à 3, dans lequel un dispositif de détection de cible à identifier (185, 186) permettant de détecter la présence ou l'absence de ladite cible (200) à identifier est disposé dans une position éloignée de ladite rangée d'éléments récepteurs de lumière.

5. Le dispositif d'identification personnelle (1) selon la revendication 4, dans lequel ledit dispositif de détection de cible à identifier (185, 186) est disposé en plusieurs exemplaires, une vitesse de ladite cible (200) à identifier est calculée à partir d'une différence entre les temps de passage d'une extrémité de ladite cible (200) à identifier, qui sont détectés par ladite pluralité de dispositifs de détection de cible à identifier (185, 186), et la correction de distance de ladite image dans une direction de balayage est réalisée sur la base de la vitesse de ladite cible (200) à identifier.

6. Le dispositif d'identification personnelle (1) selon la revendication 4, dans lequel la vitesse de ladite cible (200) à identifier est calculée à partir d'une différence entre les temps de passage d'une extrémité de ladite cible (200) à identifier, qui sont détectés par ladite rangée d'éléments récepteurs de lumière et ledit dispositif de détection de cible à identifier (185, 186) disposé en un seul exemplaire ou en plusieurs exemplaires, et une correction de distance de ladite image dans une direction de balayage est réalisée sur la base de la vitesse de ladite cible (200) à identifier.

7. Le dispositif d'identification personnelle (1) selon l'une quelconque des revendications 1 à 6, dans lequel ladite rangée d'éléments récepteurs de lumière contient une pluralité d'éléments récepteurs de lumière (11) disposés en rangée le long d'une ligne courbe.

8. Le dispositif d'identification personnelle (1) selon l'une quelconque des revendications 2 à 6, dans lequel ladite rangée d'éléments récepteurs de lumière comprend une pluralité de rangées d'éléments récepteurs de lumière (11a, 11b, 11c), et lesdites rangées d'éléments récepteurs de lumière (11a, 11b, 11c) sont disposées le long d'une ligne courbe.

9. Le dispositif d'identification personnelle (1) selon l'une quelconque des revendications 6 ou 7, dans lequel un intervalle entre deux éléments récepteurs de lumière adjacents dans ladite rangée d'éléments récepteurs de lumière est compris entre 0.02 mm et 0.5 mm.

10. Dispositif d'identification personnelle (1) selon l'une quelconque des revendications 1 à 9, comprenant un boîtier (20), ainsi qu'une source lumineuse (12) et une rangée d'éléments récepteurs de lumière (10) tous deux disposés dans ledit boîtier (20), ledit dispositif (1) fonctionnant de telle sorte que quand un doigt (200) est inséré dans ledit boîtier (20), la lumière provenant de ladite source lumineuse (12) est émise en direction du doigt (200), la lumière ayant traversé le doigt (200) est détectée par ladite rangée d'éléments récepteurs de lumière (10), et un motif de vaisseaux sanguins (201) du doigt (200) est produit à partir des signaux de sortie de ladite rangée d'éléments récepteurs de lumière (10), ce qui permet l'identification personnelle sur la base du motif de vaisseaux sanguins produit (201), dans lequel ledit boîtier (20) possède une cavité (21) dans laquelle le doigt (200) est inséré, et ladite rangée d'éléments récepteurs de lumière (10) est disposée perpendiculairement au sens de la profondeur de ladite cavité (21).

11. Dispositif d'identification personnelle (1) selon l'une quelconque des revendications 1 à 10, comprenant un élément de support en forme de C (40) incluant un premier élément (41), un second élément (42) et un troisième élément (43) permettant de relier ledit premier élément (41) et ledit second élément (42) l'un à l'autre, une source infrarouge (42) montée sur ledit premier élément (41), et une rangée d'éléments récepteurs de lumière (10) montée sur ledit second élément (42), ledit dispositif (1) fonctionnant de telle sorte que quand un doigt (200) est balayé au-dessus de ladite rangée d'éléments récepteurs de lumière (10), un rayon infrarouge provenant de ladite source infrarouge (12) est émis en direction du doigt (200), le rayon infrarouge ayant traversé le doigt (200) est détecté par ladite rangée d'éléments récepteurs de lumière (10), et un motif de vaisseaux sanguins (201) du doigt (200) est produit à partir des signaux de sortie de ladite rangée d'éléments récepteurs de lumière (10), ce qui permet l'identification personnelle sur la base du motif de vaisseaux sanguins produit.

12. Dispositif d'identification personnelle (1) selon la revendication 10, comprenant un élément inférieur, un élément de cadre disposé de manière à entourer ledit élément inférieur sur trois côtés de celui-ci, ladite source infrarouge étant montée sur ledit élément de cadre, et ladite rangée d'éléments récepteurs de lumière (10) étant montée sur ledit élément inférieur, ledit dispositif (1) fonctionnant de telle sorte que quand un doigt est balayé au-dessus de ladite rangée d'éléments récepteurs de lumière (10), un rayon infrarouge provenant de ladite source infrarouge (12) est émis en direction du doigt, le rayon infrarouge ayant traversé le doigt (200) est détecté par ladite rangée d'éléments récepteurs de lumière, et un motif de vaisseaux sanguins (201) du doigt (200) est produit à partir des signaux de sortie de ladite rangée d'éléments récepteurs de lumière (10), ce qui permet l'identification personnelle sur la base du motif de vaisseaux sanguins produit (201).

13. Dispositif d'identification personnelle (1) selon la revendication 10, ledit dispositif fonctionnant de telle sorte que quand un doigt (200) est inséré dans ledit boîtier, un rayon infrarouge provenant de ladite source infrarouge (12) est émis en direction du doigt (200), le rayon infrarouge ayant traversé le doigt est détecté par ladite rangée d'éléments récepteurs de lumière (10), et un motif de vaisseaux sanguins (201) du doigt (200) est produit à partir des signaux de sortie de ladite rangée d'éléments récepteurs de lumière (10), ce qui permet l'identification personnelle sur la base du motif de vaisseaux sanguins produit (201), dans lequel ledit boîtier possède une surface intérieure lisse pour empêcher une partie du rayon infrarouge provenant de la source infrarouge, qui a été réfléchie par le doigt, de pénétrer dans ladite rangée d'éléments récepteurs de lumière (10).

14. Le dispositif d'identification personnelle (1) selon l'une quelconque des revendications 1 à 13, dans lequel l'identification personnelle est réalisée en comparant un paramètre caractéristique préalablement enregistré et un paramètre caractéristique d'une image obtenue à partir des signaux de sortie de ladite rangée d'éléments récepteurs de lumière (10).

15. Le dispositif d'identification personnelle (1) selon la revendication 3, dans lequel ledit dispositif de détection de position est muni d'un bouton (181) pouvant être poussé par le doigt (200), un dispositif de nettoyage (183) est monté sur ledit bouton (181) et une surface de ladite rangée d'éléments récepteurs de lumière (10) est nettoyée grâce à un balayage dudit bouton (181).
